# EUROPÄISCHE PATENTSCHRIFT

(11) **EP 3 141 595 B1**
(45) Veröffentlichungstag und Bekanntmachung des Hinweises auf die Patenterteilung: **20.03.2019**
(21) Anmeldenummer: 15184910.6
(22) Anmeldetag: 11.09.2015
(51) Int. Cl.: C12M 1/107, C12M 3/00, C12M 1/12, C12M 1/00, C12M 1/34, C12M 1/02, C12M 1/33, A23N 1/00, C02F 11/00

(54) **VERFAHREN UND VORRICHTUNG ZUM ERZEUGEN VON BIOGAS**
DEVICE AND METHOD FOR CREATING BIOGAS
PROCEDE ET DISPOSITIF DESTINES A LA PRODUCTION DE BIOGAZ

(43) Veröffentlichungstag der Anmeldung: 15.03.2017
(73) Patentinhaber: pro agri gmbh, 19053 Schwerin (DE)
(72) Erfinder: Beckers, Klaus, 19053 Schwerin (DE)
(74) Vertreter: Raffay & Fleck

(56) Entgegenhaltungen:
- EP-A1- 1 669 326
- EP-A1- 2 017 230
- EP-A1- 2 239 235
- EP-A1- 2 581 439
- WO-A2-2011/079925
- WO-A2-2014/090376
- DE-A1- 19 752 371
- DE-A1- 19 813 022
- DE-A1-102007 000 834
- DE-A1-102008 041 993
- DE-U1-202004 010 707
- DE-U1-202010 012 478

## Beschreibung

Die Erfindung betrifft ein Verfahren zum Erzeugen von Biogas durch Vergärung von pumpfähigen, insbesondere auch Feststoffe enthaltenden oder mit organischen Feststoffen vermengten organischen Ausgangsmaterialien, insbesondere von Güllen und Jauchen aus der Tierhaltung, aber auch von Rückständen aus der Nahrungsmittelverarbeitung, wie z.B. Molke oder Fruchtsaftresten, sowie von anderen organischen Zwischen-, Rest- oder Abfallstoffen aus der gewerblichen Produktion oder Verarbeitung. Die Erfindung betrifft weiterhin eine Vorrichtung zum Erzeugen von Biogas durch Vergärung von pumpfähigen, insbesondere auch Feststoffe enthaltenden oder mit organischen Feststoffen vermengten organischen Ausgangsmaterialien, insbesondere solcher wie vorstehend genannt. Bereits seit mehreren Jahren wird die Erzeugung von Biogas in großem Maßstab als Quelle für alternative, CO₂-neutrale Energie genutzt. Entsprechende Biogasanlagen befinden sich insbesondere im ländlichen Raum und dienen dort sowohl der Erzeugung elektrischer Energie als auch der Generation von nutzbarer Wärme, dies häufig in Kraft-Wärme-Kopplung. In der Regel sind sie mit Blockheizkraftwerken verbunden, die in Biogasmotoren das entstehende Biogas verbrennen und dabei einen Generator zur Stromerzeugung antreiben. Die gleichzeitig entstehende Abgas- und Kühlwasserwärme des Biogasmotors wird als Nutzwärme ausgekoppelt. Die bestehenden landwirtschaftlichen Biogasanlagen gewinnen ihr Biogas überwiegend aus eigens dafür angebauter pflanzlicher Biomasse, insbesondere aus Silomais. Soweit dann auch Gülle aus der Tierhaltung vorhanden ist, wird diese in den Silomais-Biogasanlagen mitvergoren.

Über ausreichende Ressourcen an Ackerflächen zur Erzeugung der erforderlichen pflanzlichen Biomasse verfügen jedoch in der Regel nur große Ackerbaubetriebe. Die Mehrzahl der kleinen und mittleren familienbäuerlichen Landwirtschaftsbetriebe verfügt nicht darüber. Gerade aber in den vielen kleinen und mittleren Betrieben mit Tierhaltung, aber auch in reinen gewerblichen Tierhaltungsbetrieben ohne Ackerbau, fällt insgesamt eine sehr hohe Menge an Gülle und Mist an. Dieses mengenmäßig bedeutsame, ohnehin anfallende und kostenlose Potential an Substrat zur Erzeugung erneuerbarer Energie bleibt vielfach ungenutzt, da
- kein ausreichend leistungsfähiges und damit kein wirtschaftlich wettbewerbsfähiges Biogasverfahren für die Vergärung allein von Gülle bzw. von Substraten mit hohen Gülleanteilen existiert,
- keine geeignete, ausreichend leistungsfähige und damit keine wirtschaftlich wettbewerbsfähige Anlagentechnik im Bereich kleinerer und mittlerer Biogasanlagen (insbesondere 25 bis 250 KW elektrisch) existiert.

DE202004010707 U offenbart eine Vorrichtung zur gleichzeitigen anaerobe Behandlung von Abwässern und organischen Feststoffen. Die Vorrichtung zur Vergärung gemäß D1 umfasst eine äußere Kammer (1), die mit der inneren Kammer (2) über eine Trennvorrichtung, vorzugsweise als Siebblech ausgebildet, in Verbindung steht. Somit wird erreicht, dass die Feststoffpartikel der zu vergärenden Biomasse in der äußeren Kammer zurückgehalten werden.

Dieser Lücke widmet sich die vorliegende Erfindung, die es sich zur Aufgabe gemacht hat, ein Verfahren und eine Vorrichtung zum Erzeugen von Biogas durch Vergärung von pumpfähigen, gegebenenfalls Feststoffe enthaltenden oder mit organischen Feststoffen vermengten organischen Ausgangsmaterialien zu schaffen, welche den Betrieb von leistungsfähigen, kompakten und zu günstigen Investitionskosten herzurichtenden Anlagen zur Biogasgewinnung erlauben.

Diese Aufgabe wird hinsichtlich des Verfahrensaspektes gelöst durch ein Verfahren zum Erzeugen von Biogas durch Vergärung von pumpfähigen, organischen Ausgangsmaterialien, insbesondere von Güllen oder Jauchen aus der Tierhaltung, mit den Merkmalen des Anspruches 1. Vorteilhafte Weiterbildungen dieses erfindungsgemäßen Verfahrens sind in den abhängigen Ansprüchen 2 bis 8 bezeichnet. Der vorrichtungsseitige Aspekt der erfindungsgemäßen Lösung ist im Anspruch 9 festgehalten. Vorteilhafte Weiterbildungen der erfindungsgemäßen Vorrichtung sind in den Ansprüchen 10 bis 13 genannt.

Das erfindungsgemäße Verfahren zum Erzeugen von Biogas durch Vergärung von pumpfähigen organischen Ausgangsmaterialien, insbesondere von Güllen oder Jauchen aus der Tierhaltung, zeichnet sich dabei durch folgende Merkmale und Verfahrensschritte aus:
- Ein Zustrom des organischen Ausgangsmaterials, das typischerweise in substantiellem Anteil auch gröbere organische Feststoffe enthält, wird separiert in einen dünnflüssigen Dünnstoffanteil, der Feststoffpartikel in einem gegenüber dem Ausgangsmaterial geringeren Umfang und in der Regel mit überwiegend geringeren Partikelgrößen enthält, und dabei in der Regel auch einen deutlich niedrigeren Trockensubstanzgehalt (hier wie nachfolgend bezieht sich die Angabe Trockensubstanzgehalt stets auf einen Anteil an Trockensubstanz je Volumen- oder Masseeinheit) aufweist, und in einen Dickstoffanteil, der einen verglichen mit dem Ausgangsmaterial, somit auch mit dem Dünnstoffanteil deutlich höheren Anteil an Feststoffpartikeln, dabei in der Regel auch solchen mit größeren Partikelgrößen, enthält, dabei dann in der Regel auch einen deutlich höheren Trockensubstanzgehalt aufweist, und der typischerweise eine gegenüber dem Ausgangsmaterial höhere Viskosität, dabei eine eher breiige bis stichfeste Konsistenz hat. Der Dünnstoffanteil ist dabei massen- und volumenmäßig regelmäßig wesentlich größer als der Dickstoffanteil. Der Dünnstoffanteil ist eine feine Suspension und dünnflüssig. Dabei wird der Dünnstoffanteil bestimmt durch den Anteil, den eine Separationseinrichtung, die Durchlassöffnungen von bestimmter Öffnungsweite hat passieren lässt. Der von der Separationseinrichtung zurückgehaltene Anteil bildet dann den Dickstoffanteil. Die Separationseinrichtung, die z.B. mit einem oder mehreren Siebeinsatz/Siebeinsätzen ausgerüstet sein kann, hat in der im Rahmen der Erfindung vorgesehenen Anwendung typischerweise Durchlassöffnungen mit Öffnungsweiten von 20*µ*m bis 2 mm. Insbesondere liegen diese Öffnungsweiten von 50 *µ*m bis 1 mm. Besonders bevorzugt werden Öffnungsweiten von 100 *µ*m bis 500 *µ*m. Vielfach werden insbesondere Öffnungsweiten von 200 *µ*m bis 300 *µ*m gewählt werden, zum Teil auch nur bis 250 *µ*m.
   Der Trockensubstanzgehalt des Dünnstoffanteils beträgt dabei in der Regel nicht mehr als 10 Gew.-%, liegt häufig sogar bei ≤ 5 Gew.-%. Die Viskosität des Dünnstoffanteils ist vergleichsweise niedrig. Im Gegensatz zu dem Dünnstoffanteil ist der massen- und volumenmäßig wesentlich kleinere Dickstoffanteil im Vergleich zum Ausgangsmaterial verdickt und enthält einen deutlich erhöhten Feststoffanteil, in dem nahezu alle gröberen Feststoffpartikel, die in dem Ausgangsmaterial vorhanden waren, enthalten sind. Dabei enthält der Dickstoffanteil regelmäßig auch einen verglichen mit dem Dünnstoffanteil deutlich höheren Trockensubstanzgehalt. In der Regel liegt der Trockensubstanzgehalt des Dickstoffanteils dabei oberhalb von 10 Gew.-%, zumeist im Bereich von 15-25 Gew.-% oder sogar noch darüber (insbesondere, wenn dem Dickstoffanteil noch weitere organische Feststoffe beigemengt werden, kann sich der Trockensubstanzgehalt noch weiter erhöhen).
- Der Dünnstoffanteil wird einem Dünnstofffermenter zugeführt. In diesem Dünnstofffermenter sind Mikroorganismen auf in dem Füllvolumen in der Schwebe gehaltenen Aufwuchskörpern aufgewachsen, mit deren Hilfe der Dünnstoffanteil in dem Dünnstofffermenter fermentiert wird. Die Aufwuchskörper können in dem Dünnstofffermenter, insbesondere auch unterstützt durch eine Rühr- oder interne Umwälzeinrichtung, in der Schwebe gehalten werden, indem sie aus einem Material bestehen, das in seiner Dichte unter Berücksichtigung des Aufwuchses in etwa der Dichte von Wasser entspricht. Weiterhin kann auch eine externe Umwälzung des in dem Dünnstofffermenter enthaltenen Dünnstoffanteils dazu beitragen, die Aufwuchskörper in dem Volumen des Dünnstofffermenters zu verteilen (also in der Schwebe zu halten). Der Dünnstofffermenter ist dabei auch als eine Fermenterkammer ausgebildet, die abgeschlossen ist und den Dünnstoffanteil aufnimmt.
- In dem Dünnstofffermenter entstehendes Biogas wird aufgefangen, z.B. in einem Biogas-Speichersack oder einem Membrangasspeicher, und für eine weitere Verwendung abgeführt.
- In dem Dünnstofffermenter entstehende Gärreste werden abgeführt.

In der industriellen Abwasserbehandlung ist der Einsatz von im zu behandelnden, mit organischer Fracht versetzten Abwasser schwebenden Aufwuchskörpern zwar noch nicht weithin genutzter Stand der Technik, aber dennoch, wenn auch eher aus der aeroben Vergärung und der Denitrifikation von Abwässern grundsätzlich bekannt. Hinsichtlich Menge und Qualität der organischen Fracht lässt sich Gülle jedoch nicht mit industriellen Abwässern vergleichen. Bei Gülle liegt der Gehalt an organischer Trockensubstanz je m³ nicht nur bis zu 10-fach höher, insbesondere Rindergülle und Mastschweinegülle enthalten auch vergleichsweise hohe Feststoffanteile mit partikulär gebundener organischer Substanz, während in industriellen Abwässern die organische Fracht häufig überwiegend gelöst vorliegt, was für einen Abbau durch Verstoffwechselung erheblich einfacher ist. Hohe Feststoffanteile - wie in Gülle - bedeuten darüber hinaus immer eine hohe Gefahr für das Verblocken (Zusetzen) der Aufwuchskörper durch die Feststoffpartikel, mit der Folge von starker Einschränkung bis hin zum Verlust der Funktionsfähigkeit der Aufwuchskörper und damit des gesamten Prozesses. Zusätzlich erhöhen hohe Feststoffanteile im Gärmedium die Viskosität des Gärmediums, was ebenfalls nachteilig für den Gärprozess ist.

Aus diesen Gründen besteht ein wesentliches, die Effizienz des Verfahrens steigerndes Moment in dem Abtrennen eines Dünnstoffanteils und in der Kombination einer Ansiedlung von Mikroorganismen in dem Dünnstofffermenter auf Aufwuchskörpern.

Die Ansiedlung der Mikroorganismen auf Aufwuchskörpern führt im Dünnstofffermenter nicht nur zu maximaler Kontaktfläche zwischen Mikroorganismen und Nährstoffen und wesentlich erhöhter Populationsdichte der Mikroorganismen, gleichzeitig wird durch die Immobilisierung bzw. "Fixierung" der Mikroorganismen ein Ausschwemmen der Mikroorganismen mit dem ausgetragenen Gärrest verhindert. Dadurch kann die Verweilzeit des Gärmediums im Dünnstofffermenter schadlos kürzer sein als die Dauer des Regenerationszyklus insbesondere der Methanbakterien, die in der Regel ca. 15 Tage beträgt. In herkömmlichen Biogasanlagen sind Kontaktfläche und Populationsdichte ganz erheblich niedriger, Mikroorganismen werden dort kontinuierlich mit dem Gärrest ausgeschwemmt und die Verweilzeit muss zwangsläufig oberhalb der Dauer des Regenerationszyklus insbesondere der Methanbakterien liegen, was konsequent proportional höheres Fermentervolumen und niedrigere Faulraumproduktivität bedeutet.

Für das vorliegende Verfahren können beispielsweise entsprechende Aufwuchskörper von dem schwedischen Unternehmen AnoxKaldnes Verwendung finden. Auch können z.B. die von der Multi Umwelttechnologie AG aus Aue/Sachsen, Deutschland unter der Markenbezeichnung Mutag Biochip ® angebotenen Aufwuchskörper eingesetzt werden oder gleichwertige Aufwuchskörper, die einen Rückhalt der Mikroorganismen durch Besiedlung und Aufwuchs auf den Aufwuchskörpern gewährleisten.

Mit Vorteil kann der bei der Separation anfallende Dickstoffanteil einem Dickstofffermenter zugeführt und dort jedenfalls zum Teil auch fermentiert werden. In einem solchen Dickstofffermenter wird der Dickstoffanteil aber auch - in der Regel vor allem - hydrolisiert und versäuert. Bevor der Dickstoffanteil in den Dickstofffermenter eingetragen wird, kann er, bzw. können die darin enthaltenen Feststoffe, zerkleinert, aufgebrochen und/oder homogenisiert werden, z.B. in einer Kugelmühle. Dabei können dem Dickstoffanteil z.B. noch zusätzliche Feststoffe, wie z.B. Mist, Gras, Stroh oder andere Biomasse, und/oder Prozesshilfsmittel, wie hydrolysefördernde Enzyme, Spurenelemente oder ähnliches, und/oder auch ein Rezirkulat aus dem Dickstoff- oder auch aus dem Dünnstofffermenter beigemengt und mit zerkleinert, aufgebrochen und/oder homogenisiert werden. Dabei kann auch bereits eine Vorerwärmung erfolgen, indem z.B. eine verwendete Kugelmühle beheizt ist. Ein nach gewisser Zeitdauer hydrolysierter Anteil wird, wenn ein Dickstofffermenter in der oben beschriebenen Weise eingesetzt wird, aus dem Dickstofffermenter abgezogen und - nach Durchlaufen eines Zerkleinerungs- und/oder Aufbrech- und/oder Homogenisierungsschrittes, in einer elektrokinetischen Desintegration - dem Dünnstofffermenter zugeführt bzw. dem Dünnstoffanteil beigemengt oder dem Zustrom organischen Ausgangsmaterials zugegeben. In dem Dickstofffermenter entstehendes Biogas wird dabei ebenfalls aufgefangen und für eine weitere Verwendung abgeführt, ggf. einem Biogasspeicher zugeleitet. Hierbei kann insbesondere das in dem Dickstofffermenter entstehende Biogas mit dem in dem Dünnstofffermenter entstehenden Biogas zusammengeführt werden. Eine solche getrennte und parallel geführte Behandlung des Dickstoffanteils in einem Dickstofffermenter trägt weiter zu Effizienzsteigerung des Verfahrens bei. Zwar ist die Dauer der Behandlung des Dickstoffanteils in dem Dickstofffermenter länger anzusetzen als die Verweilzeit des Dünnstoffanteils im Dünnstofffermenter. Jedoch ist der Dickstoffanteil volumenmäßig deutlich kleiner, so dass auch der Dickstofffermenter mit einem vergleichsweise geringen Volumen auskommt. Der hydrolysierte und angesäuerte Anteil aus dem Abstrom aus dem Dickstofffermenter kann nun wie der zuvor separierte Dünnstoffanteil in dem Dünnstofffermenter mit kurzer Verweildauer sehr effizient zur Biogasgewinnung vergoren werden.

Ein Anteil des Füllvolumens rwirdaus dem Dünnstofffermenter einer Behandlung zum Zerkleinern, Aufbrechen und/oder Homogenisieren der darin enthaltenen Feststoffe unterzogen, einer elektrokinetischen Desintegration, und nach dieser Behandlung, wieder dem in dem Dünnstofffermenter vergärenden Volumen zugeführt werden. Dies kann einmalig für eine bestimmte Behandlungsdauer geschehen, chargenweise und mit zeitlichen Abständen, aber auch - und dies mit Vorteil - kontinuierlich oder semikontinuierlich. Dabei kann ein solcher kontinuierlicher oder semikontinuierlicher Volumenstrom auch zum (externen) Umwälzen des in dem Dünnstofffermenter enthaltenen Dünnstoffanteils ausgenutzt werden. Nach der Behandlung zum Zerkleinern, Aufbrechen und/oder Homogenisieren der in dem Volumenstrom enthaltenen Feststoffe, also nach der elektrokinetischen Desintegration, wird der so behandelte Anteil des Füllvolumens wieder in den Dünnstofffermenter gegeben. Bei diesem Vorgehen werden die Aufwuchskörper mittels einer Rückhaltevorrichtung, z.B. Rückhalterechen oder -sieb, in dem Dünnstofffermenter zurückgehalten, so dass verhindert wird, dass auch diese die Behandlung, durch die elektrokinetische Desintegration, durchlaufen. So wird durch die Lokalisierung der Mikroorganismen auf den Aufwuchskörpern und das Zurückhalten der Aufwuchskörper, und damit der darauf angesiedelten Mikroorganismen von der Behandlung, der elektrokinetischen Desintegration, verhindert, dass die für den Fermentationsprozess entscheidenden Mikroorganismen nicht in diesem Prozessschritt abgetötet werden. Unter einer als eine Möglichkeit der entsprechenden Behandlung hier angeführten elektrokinetischen Desintegration wird die Zerkleinerung bzw. Auflösung von bestehenden Strukturen mittels an einer von dem zu desintegrierenden Medium umspülten Elektrode angelegten elektrischen Hochspannung verstanden. Die dabei auftretenden elektrischen Kräfte im Hochspannungsfeld verformen und destabilisieren die Zellwände und - membranen. Übersteigt die Verformung dabei die elastische Widerstandskraft der Zellwände bzw. -membranen, werden diese durchlässig und die Zellinhaltsstoffe werden freigesetzt, liegen dann unmittelbar für die Verstoffwechselung durch die Mikroorganismen und damit die Biogaserzeugung frei bzw. vor. Auch größere Agglomerate von Feststoffpartikeln werden hierbei aufgelöst und "gecrackt". Entsprechende elektrokinetische Desintegrationseinrichtungen sind bereits auf dem Markt erhältlich, z.B. das System BioCrack® der Hugo Vogelsang Maschinenbau GmbH aus Essen/Oldenburg, Deutschland. Es ist hier aber wichtig zu betonen, dass die Behandlung zum Zerkleinern, Aufbrechen und/oder Homogenisieren der mitgeführten Feststoffe auch in anderer Weise geschehen kann, z.B. mechanisch in einer Mühle oder einem Messerzerkleinerer. An dieser Stelle ist wichtig zu betonen, dass die oben beschriebene Behandlung keine Anordnung einer entsprechenden Vorrichtung außerhalb des Dünnstofffermenters voraussetzt. Die Behandlung kann z.B. auch in einem Abschnitt des Fermentervolumens des Dünnstofffermenters selbst erfolgen, der z.B. mit einem Käfig von dem Bereich mit den Aufwuchskörpern abgetrennt sein kann.

Gerade eine solche Kombination mit der optionalen und vorteilhaften Behandlung zum Zerkleinern, Aufbrechen und/oder Homogenisieren des Feststoffanteils in dem Inhalt des Dünnstofffermenters, in Form der elektrokinetischen Desintegration, sorgt nun aber dafür, dass das Potential der erheblich gesteigerten Abbauintensität im Dünnstofffermenter noch weiter ausgeschöpft werden kann. Das, insbesondere kontinuierliche oder semikontinuierliche, auch mehrfache, "Cracken" der vergleichsweise hohen Partikelfrachten bei z.B. Gülle sorgt dafür, dass nach der entsprechenden Behandlung, durch elektrokinetische Desintegration, die wertvollen organischen Bestandteile besser verwertbar und auch in höherem Gesamtmaße vorliegen und damit einfacher, mit höherer Biogasausbeute und schneller von den Mikroorganismen verstoffwechselt werden können. Je stärker durch die Behandlung, durch die elektrokinetische Desintegration, die Feststoffpartikel (-agglomerationen) zertrümmert und verkleinert werden und gleichzeitig die Viskosität des Gärmediums verringert wird, desto weniger groß ist gleichzeitig die Gefahr des Verblockens der Aufwuchsträger, für die dauerhafte Prozessstabilität ein äußerst förderlicher Punkt. Gerade der Umstand, dass die Mikroorganismen, die auf den Aufwuchskörpern angesiedelt sind, effizient im Inneren des Dünnstofffermenters zurückgehalten werden können, erlaubt hier die Durchsetzung eines vergleichsweise großen Volumenstromes durch die Behandlung zum Zerkleinern, Aufbrechen und/oder Homogenisieren, durch eine elektrokinetische Desintegration, der deutlich über einem Volumenstrom liegt, der bei einem Fermenter, in dem die Mikroorganismen frei in der Suspension schweben, realisiert werden könnte. Denn durch diese Behandlung, z.B. die elektrokinetische Desintegration, wird nicht nur die von den Mikroorganismen zu verstoffwechselnde organische Fracht des organischen Ausgangsmaterials (des sogenannten Substrates) verbessert aufgeschlossen, sondern es werden auch die Zellen der nützlichen und in dem Fermenter grundsätzlich erwünschten Mikroorganismen aufgebrochen und zerstört, so dass es hier, wenn keine Möglichkeit des Rückhaltes der Mikroorganismen gegeben ist, zu einem Abtöten eines zu großen Anteils der Mikroorganismen und damit einem Erlahmen bis hin zum Stillstand des Gärprozesses kommen würde.

Die oben beschriebene - insbesondere kontinuierlich oder semikontinuierlich durchgeführte - Behandlung zum Zerkleinern, Aufbrechen und/oder Homogenisieren des Feststoffanteils in dem Inhalt eines mit auf Aufwuchskörpern angesiedelten Mikroorganismen bestückten Dünnstofffermenters, insbesondere durch elektrokinetische Desintegration, kann auch für sich genommen auf eine eigenständig erfinderische Weise und ohne eine Separation des Ausgangsmaterials in Dick- und Dünnstoffanteil durchgeführt werden, insbesondere dann, wenn das organische Ausgangsmaterial per se schon dünnflüssig genug ist, und nur wenig grobe Partikelfracht enthält, um so als "Dünnstoff" klassifiziert zu werden (z.B. bei Molke, Zuchtsauengülle, Gülle aus Aquakulturen oder dergleichen).

Um für das Aufheizen auf die üblicherweise gegenüber einer normalen Umgebungstemperatur erhöhte Temperatur, bei der die Fermentierung erfolgt, erforderliche Fermenterheizenergie zu sparen, wird mit Vorteil der Zustrom organischen Materials über Wärmetausch mit abströmenden Gärresten vorerwärmt. Hierfür können übliche Wärmetauscher, insbesondere Gegenstromwärmetauscher, zum Einsatz kommen.

Während einige organische Ausgangsstoffe, wie z.B. Zuchtsauengülle, Fisch- und Krustentiergüllen aus Aquakulturen und ggfs. auch Molke, in der Regel weit überwiegend lediglich feine Feststoffpartikel geringer Größe und nur vergleichsweise wenig grob-partikuläre Fracht enthalten und damit insgesamt als Dünnstoffanteil behandelt werden können, erfüllen die klassischen, vom mengenmäßigen Aufkommen bedeutsamen landwirtschaftlichen Ausgangsstoffe, insbesondere Rindergülle und Mastschweinegülle diese Forderung nicht. Da diese in erheblichem Umfang gröbere und insgesamt mehr Feststoffpartikel enthalten können sie in der Regel nicht geschlossen als Dünnstoffanteil behandelt werden, so dass die eingangs des Verfahrens durchgeführte Separation notwendig ist. Sollte das Ausgangsmaterial bereits in einer vollständig als Dünnstoff zu qualifizierenden Form vorliegen, so kann die Separation entfallen, die Behandlung des gesamten Ausgangsmaterials ausschließlich in dem Dünnstofffermenter vollzogen werden.

Wie oben schon erwähnt, ist es grundsätzlich möglich, dem Zustrom organischen Ausgangsmaterials weitere organische Feststoffe zuzusetzen, z.B. Mist, Gras, Stroh, Hühnertrockenkot oder anderes. Diese zusätzlichen Feststoffe können dabei dem gesamten organischen Ausgangsmaterial, dem Dünnstoffanteil oder aber auch - sofern vorhanden - dem Dickstoffanteil beigemengt werden. Diese organischen Feststoffe werden dabei typischerweise gezielt vorzerkleinert und können ggf. durch Zugabe von Flüssigkeit angemaischt oder in ihrer Viskosität angepasst werden, damit sie pumpfähig sind. Die Vorzerkleinerung kann dabei z.B. in einem Feststoffvorlagebehälter erfolgen und als Feinzerkleinerung in einer Kugelmühle fortgesetzt werden, in der ebenfalls dem Material Rezirkulat aus dem Prozess oder beispielsweise auch Wasser zum Anmaischen oder zur Absenkung der Viskosität beigegeben werden können.

Um die Fermentationsgeschwindigkeit zu erhöhen und den Mikroorganismen das organische Material in einer besser aufbereiteten Form zu präsentieren, kann bereits der Zustrom des organischen Ausgangsmaterials, z.B. vor der Aufteilung in den Dickstoffanteil und den Dünnstoffanteil, aber auch in Form eines bereits anfänglich ausschließlich gegebenen Dünnstoffanteils, einer Behandlung zum Zerkleinern, Aufbrechen und/oder Homogenisieren, durch elektrokinetischen Desintegration, unterzogen werden. Auf diese Weise werden bereits an dieser Stelle die Ausgangsbestandteile des organischen Materials in entsprechender Weise behandelt, insbesondere desintegriert: es werden Agglomerationen gelöst, Zellinhaltstoffe freigesetzt, die dann als Nährstoffe für die Mikroorganismen besser und schneller zugänglich vorliegen, und die Viskosität wird verringert. Auch kann so der besonders effizient zu vergärende Dünnstoffanteil erhöht werden. Weiterhin ist es auch möglich, zumindest einen Anteil des Abzugs aus einem ggf. vorhanden Dickstofffermenter einer Behandlung zum Zerkleinern, Aufbrechen und/oder Homogenisieren, z.B. einer elektrokinetischen Desintegration, zu unterziehen und den so insbesondere desintegrierten Anteil anschließend dem Dünnstofffermenter einzuspeisen oder in den Zustrom organischen Ausgangsmaterials mit einzuleiten. Auf diese Weise kann eine weitere Fermentierung des Abzugs aus dem Dickstofffermenter erfolgen, so dass auch hier eine Effizienzsteigerung und eine Erhöhung der Biogasausbeute sowie eine Beschleunigung zu erzielen sind. Für die mögliche Behandlung zum Zerkleinern, Aufbrechen und/oder Homogenisieren, z.B. eine elektrokinetische Desintegration, des Inhalts des Dünnstofffermenters einerseits und die optional durchzuführende entsprechende Behandlung, z.B. elektrokinetische Desintegration, des organischen Ausgangsmaterials und/oder die ebenfalls optional durchzuführende entsprechende Behandlung, z.B. elektrokinetische Desintegration, des Abzugs aus dem ggf. vorhandenen Dickstofffermenter kann ein und derselbe Anlagenteil, also z.B. ein und dieselbe elektrokinetische Desintegrationseinrichtung, verwendet werden. Hierzu sind dann entsprechende Leitungssysteme und -verteiler vorzusehen, die über entsprechende Ventilschaltungen eine entsprechende Verbindung mit der Behandlungseinrichtung, z.B. elektrokinetischen Desintegrationseinrichtung, erlauben. In einem solchen Fall kann dann natürlich die Behandlung, z.B. elektrokinetische Desintegration, des Inhalts des Dünnstofffermenters nicht durchgehend kontinuierlich erfolgen, sondern allenfalls nur semikontinuierlich, indem jedenfalls zu den Zeiten, zu denen die Behandlungseinrichtung, z.B. elektrokinetische Desintegrationseinrichtung, in die jeweils anderen Leitungsläufe (den Zustrom organischen Ausgangsmaterials und/oder den Abzug aus einem ggf. vorhanden Dickstofffermenter) geschaltet ist, die entsprechende Behandlung, z.B. elektrokinetische Desintegration, des Inhalts des Dünnstofffermenters vorübergehend unterbrochen wird.

Das in dieser Weise in der Kombination der erfindungsgemäßen Schritte und Maßnahmen gestaltete Verfahren erlaubt insbesondere eine erheblich beschleunigte Vergärung des volumenmäßig regelmäßig wesentlich größeren Dünnstoffanteils. Die Verweilzeit sinkt hier auf die Dauer von bis zu weniger als 1 Tag, verglichen mit einer Verweilzeit von wenigstens 20 bis eher 30 Tagen des nicht separierten Ausgangsmaterials in einer herkömmlichen landwirtschaftlichen Biogasanlage. Für Ausgangsmaterialien mit hohem, für die Biogaserzeugung nutzlosem Wassergehalt, wie insbesondere Güllen und Jauchen, ist dies von ganz wesentlicher Bedeutung. Durch die spezielle Ausgestaltung ist das Verfahren gleichzeitig hoch effizient hinsichtlich der abgebauten Trockensubstanz, der Biogasausbeute und des Rest-Trockensubstanzgehaltes im Gärrest.

Da einerseits der volumenmäßig wesentlich größere Dünnstoffanteil mit ganz erheblich geringerer Verweilzeit vergoren werden kann und andererseits der eine längere Verweilzeit beanspruchende Dickstoffanteil volumenmäßig auf das notwendige Maß beschränkt wird, kann die Vergärung so auch in sehr kompakten und kleindimensionierten Vorrichtungen ein- und umgesetzt werden.

Die zunächst gestaltete Auftrennung des Zustromes des organischen Ausgangsmaterials in Dünnstoffanteil und Dickstoffanteil sowie die getrennte Behandlung dieser Fraktionen in eigenständigen Fermentern oder Fermenterkammern, dem optional vorzusehenden Dickstofffermenter (auch einer Dickstofffermenterkammer) und dem Dünnstofffermenter (auch einer Dünnstofffermenterkammer), erlauben eine spezifische Aufteilung und auf die jeweiligen Ausgangsbedingungen angepasste Behandlung mit entsprechenden Mikroorganismen und einer entsprechenden Gestaltung des Fermenters, insbesondere der Art der Besiedlung mit den Mikroorganismen und auch des jeweiligen Rühr- bzw. Mischwerkes, mit dem der jeweilige in dem zugehörigen Fermenter befindliche Anteil (Dick- bzw. Dünnstoffanteil) homogenisiert wird.

Hierin unterscheidet sich das erfindungsgemäße Verfahren erheblich von den in den herkömmlichen Biogasanlagen ablaufenden Verfahren, in denen in der Regel stets die gesamte Biomasse en bloc behandelt wird.

Das erfindungsgemäße Verfahren zum Erzeugen von Biogas ist insbesondere für die Nutzung von Güllen und Jauchen als Ausgangsmaterial (Substrat) geeignet und vorgesehen. Aufgrund der hohen Effizienz, mit der dieses Verfahren arbeitet, ist es sogar für die Verwendung von reiner Schweinegülle als Substrat geeignet, einem Ausgangsmaterial, das in der herkömmlichen Anlagentechnik als Monosubstrat größere Schwierigkeiten in der Umsetzung zu Biogas bereitet und nur beigemischt werden kann bzw. mit weiterem organischen Ausgangsmaterial gestreckt werden muss, um dann verarbeitet und vergoren bzw. fermentiert zu werden.

Während es zwar grundsätzlich möglich ist, das erfindungsgemäße Verfahren mit mesophilen Mikroorganismen bei einer Temperatur von 37°C bis 42°C zu betreiben, wird bevorzugt, dass thermophile Mikroorganismen zum Einsatz kommen, wobei die Betriebstemperatur der entsprechend bestückten Fermenter vorzugsweise bei Temperaturen von 55°C bis 60°C eingestellt wird. Es können aber auch, wenn sowohl ein Dünnstofffermenter als auch ein Dickstofffermenter verwendet werden, für diese beiden unterschiedlichen Fermenter verschiedene Temperaturen eingestellt werden. So kann in einem solchen Fall z.B. der Dünnstofffermenter mesophil, der Dickstofffermenter hingegen thermophil betrieben werden, um den erforderlichen Einsatz von Wärme zum Beheizen des Dünnstofffermentervolumens zu reduzieren. Diese unterschiedliche Temperierung kann z.B. durch gezielte Wärmezufuhr an unterschiedlichen Stellen der Vorrichtung bzw. an unterschiedlichen Anlagenteilen erfolgen.

Bei dem erfindungsgemäßen Verfahren liegt, wie bereits erwähnt, die Verweilzeit im Dünnstofffermenter bei ausgesprochen niedrigen Werten bis weniger als 1 Tag. Eine solch kurze Verweildauer und damit ein entsprechend hoher und schneller Durchsatz an organischem Ausgangsmaterial ist insbesondere deshalb möglich, da das erfindungsgemäße Verfahren sich als hoch effizient in der Umsetzung des organischen Ausgangsmaterials zu Biogas erwiesen hat. Die hohe Faulraumproduktivität und die kurze Verweilzeit ermöglichen äußerst kompakte Fermenter. Herkömmliche Biogasanlagen sind auf erheblich größere Fermenter angewiesen. Die Verweilzeit für Gülle liegt in herkömmlichen Biogasanlagen im Bereich von wenigstens 20 bis eher 30 Tagen.

In einem weiteren Aspekt gibt die Erfindung eine Vorrichtung gemäß Anspruch 9 zum Erzeugen von Biogas durch Vergärung von pumpfähigem, insbesondere auch Feststoffe enthaltendem organischen Ausgangsmaterial an. Diese Vorrichtung enthält erfindungsgemäß:
- eine Separationseinrichtung zum Aufteilen von zuströmendem organischen Ausgangsmaterial in einen dünnflüssigen Dünnstoffanteil mit einem geringen und überwiegend feinteiligen Anteil an Feststoffpartikeln und typischerweise vergleichsweise niedrigem Trockensubstanzgehalt und in einen eher breiigen bis stichfesten Dickstoffanteil mit höherem Feststoffpartikelanteil, der vor allem auch grobe Feststoffpartikel enthält, und mit typischerweise vergleichsweise hohem Trockensubstanzgehalt. Der Dünnstoffanteil, auf den in der Vorrichtungsbeschreibung Bezug genommen wird, entspricht in seinen Eigenschaften generell demjenigen, wie er im Rahmen der vorstehenden Verfahrensbeschreibung näher erläutert worden ist. Insbesondere werden dessen Eigenschaften (wie auch die Eigenschaften des Dickstoffanteils) wesentlich durch die in der wie oben beschriebenen Wahl vorgenommene Separation mit der Separationseinrichtung mit vorgegebenen Öffnungsweiten erhalten.
- einen Dünnstofffermenter zum Fermentieren des Dünnstoffanteils. In dem Dünnstofffermenter sind erfindungsgemäß Aufwuchskörper für das Aufwachsen von Mikroorganismen angeordnet, insbesondere schweben diese in dem Volumen des in dem Dünnstofffermenter enthaltenen Dünnstoffanteils in ungefähr gleichmäßiger Verteilung frei. Weiterhin kann der Dünnstofffermenter eine Rühr- und/oder Umwälzeinrichtung zum Homogenisieren des Füllvolumens des Dünnstofffermenters und zum in der Schwebe Halten der Aufwuchskörper aufweisen.

Mit Vorteil kann in der Vorrichtung auch ein Dickstofffermenter zum Hydrolisieren, Versäuren und Fermentieren des Dickstoffanteils vorgesehen sein, wobei der Dickstofffermenter insbesondere eine Mischeinrichtung zum Durchmischen des in dem Dickstofffermenter aufgenommenen Füllvolumens aufweisen kann. Der Dickstofffermenter kann insbesondere über mehrere, vorzugsweise in unterschiedlicher Höhe positionierte, Abzüge verfügen, um Schwimmschichten, Sinkschichten und Substratflüssigkeit jeweils gezielt aus dem Dickstofffermenter abziehen zu können.

Die Vorrichtung weist ferner auch noch folgende Elemente auf:
- eine Behandlungseinrichtung zum Zerkleinern und/oder Aufbrechen und/oder Homogenisieren von in dem Dünstoffanteil enthaltenen Feststoffen, als eine elektrokinetische Desintegrationseinrichtung, die eine mit dem Dünnstofffermenter verbundene Zuführung, über die ein in dem Dünnstofffermenter enthaltener Inhalt aus dem Dünnstofffermenter der Behandlungseinrichtung zugeführt werden kann, und eine mit dem Dünnstofffermenter verbundene Abführung, über die der einer entsprechenden Behandlung, z.B. einer elektrokinetischen Desintegration, unterzogene Inhalt dem Füllvolumen in dem Dünnstofffermenter wieder zugeführt werden kann, aufweist und
- eine im Bereich der Zuführleitung angeordnete Rückhaltevorrichtung zum Verhindern eines Vordringens der Aufwuchskörper in die Behandlungseinrichtung.

Die Behandlungseinrichtung, als eine elektrokinetische Desintegrationseinrichtung, kann dabei außerhalb des Dünnstofffermenters angeordnet und mit dem Innern dieses Fermenters über Rohrleitungen verbunden sein. Sie kann aber auch in dem Volumen des Dünnstofffermenters selbst angeordnet, dann z.B. über einen die Behandlungseinrichtung umgebenden Käfig vom restlichen Volumen abgetrennt sein.

Die erfindungsgemäße Vorrichtung kann zudem insbesondere modular aufgebaut sein mit einem Fermentermodul, das den Dünnstofffermenter und, sofern ein solcher vorgesehen ist, den Dickstofffermenter enthält und das insbesondere maximale Kantenabmessungen von 4 m (Breite) x 4 m (Länge) x 18 m (Höhe) aufweist, und einem Technikmodul, welches die optional vorzusehende Behandlungseinrichtung, z.B. eine elektrokinetische Desintegrationseinrichtung, wenigstens eine Pumpe, sowie eine Steuerung zum Steuern des automatisierten Betriebes der Vorrichtung aufweist und insbesondere in einem 40-Fuß-Container oder einem Gehäuse von gleicher oder geringerer Größe untergebracht ist.

Die erfindungsgemäße Vorrichtung ist mithin aufgebaut, um das erfindungsgemäße Verfahren ausführen zu können. Der - optional die - Fermenter (Dünnstofffermenter und ggf. Dickstofffermenter) kann oder können mit Vorteil als vertikal ausgerichtete Fermenterkammer(n) in einem eigenen Gehäuse untergebracht sein, welches aufgrund der Abmessungen von maximal 4 m x 4 m x 18 m, beispielsweise ca. 3,65 m x 3,65 m x 15,00 m, von einem Schwerlasttransporter (Lkw) aufgenommen und transportiert werden kann. Bereits aus den Grundabmessungen ergibt sich, dass die entsprechende(n) Fermenterkammer(n) ein nur geringes Volumen / geringe Volumina aufweist bzw. aufweisen und von daher für eine effiziente Biogaserzeugung auf eine hohe Effizienz und einen hohen Durchsatz an organischem Material angewiesen ist / sind, wie dieser durch das erfindungsgemäße Verfahren gestattet wird. Der bevorzugte modulare Aufbau der entsprechenden Vorrichtung erlaubt eine schnelle Verlegung derselben mittels z.B. Lastkraftwagen, so dass eine entsprechende Vorrichtung einfach an einen Aufstellungsort, z.B. zu einem Ort eines landwirtschaftlichen Betriebes, verbracht werden kann. Dort werden dann die entsprechenden Module, das in der Regel größte Fermentermodul und jedenfalls das vorzugsweise in einer Containergröße oder einem Gehäuse vergleichbarer Größe ausgestaltete Technikmodul, aufgestellt und entsprechend mit den Leitungsverbindungen miteinander verbunden, um dann eine funktionsfähige Vorrichtung zum Erzeugen von Biogas (eine Biogasanlage) zu erhalten.

Derartige Vorrichtungen können mithin an einem Fertigungsort gefertigt werden durch Fertigstellung der Module und müssen am Ort der Aufstellung, beispielsweise einem landwirtschaftlichen Betrieb mit Viehhaltung, lediglich aufgestellt, in den Modulen miteinander verbunden und ggf. an weitere Infrastruktureinrichtungen, z.B. eine Zuleitung zum Stromnetz, angeschlossen werden. Damit aber bleibt die erfindungsgemäße Vorrichtung auch mobil, denn sie kann in gleicher Weise schnell abgebaut werden. Dies erlaubt dann für den jeweils betroffenen potentiellen Betreiber eine andere Art der Kalkulationsbasis, indem er beispielsweise entsprechende Biogasanlagen mieten oder leasen kann, diese bei einem Verlust des Interesses an einem entsprechenden Betrieb, z.B. nach Abschaffung der Viehhaltung auf dem landwirtschaftlichen Betrieb, wieder abstoßen kann. Auch ist es möglich, eine entsprechende modular aufgebaute Anlage bzw. Vorrichtung käuflich zu erwerben, diese dann als gebrauchte Vorrichtung wieder zu veräußern, da sie in einfacher Weise abgebaut und umgezogen werden kann. Diese Möglichkeit erst ist es, die den betroffenen potentiellen Betreibern die Anschaffung einer solchen und die Investition in eine solche Vorrichtung erlauben, da sie nicht befürchten müssen, z.B. aufgrund einer wirtschaftlichen Entscheidung gegen den bisher vorhandenen Viehbestand mit einer fest am Ort errichteten Biogasanlage als "Investitionsruine" belastet zu sein.

Wenn das Ausgangsmaterial allerdings insgesamt als Dünnstoffanteil behandelt werden kann, also die Feststoffpartikelfracht nicht zu grob, bzw. der Feststoffanteil hinreichend gering ist, kann die erfindungsgemäße Vorrichtung ohne Separationseinrichtung und insbesondere auch ohne einen ansonsten vorzugsweise vorzusehenden Dickstofffermenter realisiert werden.

Weiterhin kann die Vorrichtung mit Vorteil eine Kugelmühle oder eine gleichwirkende Vorrichtung aufweisen, die - z.B. bei laufender Separation und paralleler Einspeisung des Dünnstoffanteils in den Dünnstofffermenter - den Dickstoffanteil aufnimmt, zwischenspeichert, ggfs. mit weiteren Feststoffen, Hilfsstoffen oder Rezirkulat aus dem Prozess, bzw. auch Wasser oder anderem vermengt, feinzerkleinert, homogenisiert und ggfs. vorerwärmt. Dadurch wird der Dickstoffanteil weiter aufbereitet für eine schnellere Zersetzung (Hydrolysierung) in dem optional vorgesehenen Dickstofffermenter. Wenn dem Prozess neben dem Ausgangsmaterial noch weitere Substrate zugeführt werden, kann die Vorrichtung weiterhin, also neben den vorstehend bereits beschriebenen Komponenten, noch einen Feststoffvorlagebehälter für weitere Feststoffe, ggfs. mit integrierter Vorzerkleinerung, bzw. einen Vorlagetank für weitere Flüssigkeiten umfassen.

Hier ist darauf hinzuweisen, dass die unmittelbare apparatetechnische Kombination einer Separationseinrichtung mit einem Feststoffvorlagebehälter, ggfs. mit integrierter Vorzerkleinerung, und einer Kugelmühle zu den weiter oben dargestellten verfahrenstechnischen Zwecken bei der Biogaserzeugung eine eigenständige Problemlösung und separate Erfindung darstellen kann.

Sowohl bei einer Separation des Ausgangsmaterials als auch bei einer Zuführung weiterer Feststoffe als Substrat kann z.B. das Fermentermodul neben dem Dünnstofffermenter (der Dünnstofffermenterkammer) insbesondere auch den Dickstofffermenter enthalten, der dann insbesondere als zweite Fermenterkammer in dem Modul ausgebildet ist. Das Fermentermodul enthält bei einer solchen Lösung also den Dünnstofffermenter und den Dickstofffermenter als getrennte, vertikal ausgerichtete Kammern in einem gemeinsamen Gehäuse.

Neben den beiden in einer modularen Ausgestaltung der Vorrichtung zwingend vorhandenen Modulen Fermentermodul und Technikmodul kann die erfindungsgemäße Vorrichtung noch weitere Module umfassen, so z.B. ein Biogasspeichermodul, welches wenigstens ein Gasspeicherelement aufweist und welches ebenfalls vorzugsweise in einem 40-Fuß-Container oder einem Gehäuse von gleicher oder geringerer Größe untergebracht ist, so dass auch dieses Modul mit einem Lkw straßentransportfähig ist, sowie ein Blockheizkraftwerkmodul, in dem eine Biogas-Verbrennungsmaschine und ein Generator angeordnet sind, in der Regel zur Erzeugung von elektrischem Strom und zur Gewinnung von nutzbarer Wärme.

Das erfindungsgemäße Verfahren und die erfindungsgemäße Vorrichtung können dabei nicht nur als "Stand-Alone-Lösung" verwendet und mit eigenem Substrat beschickt werden. Sie eignen sich insbesondere auch für den Einsatz zur Effizienzsteigerung und/oder Kapazitätserweiterung in bereits vorhandenen landwirtschaftlichen Biogasanlagen, in Bioabfallbiogasanlagen oder in Klärgasanlagen, indem Gärflüssigkeit aus dem Fermenter oder aus dem Nachgärbehälter einer herkömmlichen Biogasanlage, auch aus einem sogenannten Trockenfermentationsbehälter, in einer erfindungsgemäßen Vorrichtung nach dem erfindungsgemäßen Verfahren behandelt wird. Die Vorrichtung bzw. das Verfahren werden dann zur Optimierung der bereits bestehenden Biogasanlagen gleichermaßen als "Erweiterungsmodul" eingesetzt und in eine bestehende Anlage und die zugehörige Verfahrensführung integriert. Dabei kann z.B. die Separation bereits in einem Ablauf des Fermenters oder des Nachklärbeckens einer bestehenden Biogasanlage vorgenommen, der dann zurückbehaltene Dickstoffanteil in dem Fermenter oder dem Nachklärbecken belassen werden.

Weitere Vorteile und Merkmale der Erfindung ergeben sich aus der nachfolgenden Beschreibung eines Ausführungsbeispiels anhand der beigefügten Figuren. Dabei zeigen:
- Fig. 1: ein Verfahrensfließschema zur Veranschaulichung des erfindungsgemäßen Verfahrens und des Zusammenspiels der einzelnen Bestandteile einer dieses Verfahren ausführenden Vorrichtung zur Erzeugung von Biogas;
- Fig. 2: schematisch den Aufbau einer erfindungsgemäßen Vorrichtung aus unterschiedlichen Modulen; und
- Fig. 3: eine schematische Innenansicht des Fermentermoduls zur Darstellung der beiden darin nebeneinander angeordneten, vertikal ausgerichteten Fermenterkammern, der Dickstofffermenterkammer und der Dünnstofffermenterkammer.

Die Figuren, bei denen es sich um rein schematische Prinzipdarstellungen handelt, zeigen zum einen den Verfahrensablauf eines Ausführungsbeispiels des erfindungsgemäßen Verfahrens zum Erzeugen von Biogas, zum anderen schematisch den Aufbau einer erfindungsgemäßen Vorrichtung zur Erzeugung von Biogas in einem möglichen konkretisierten Ausgestaltungsbeispiel.

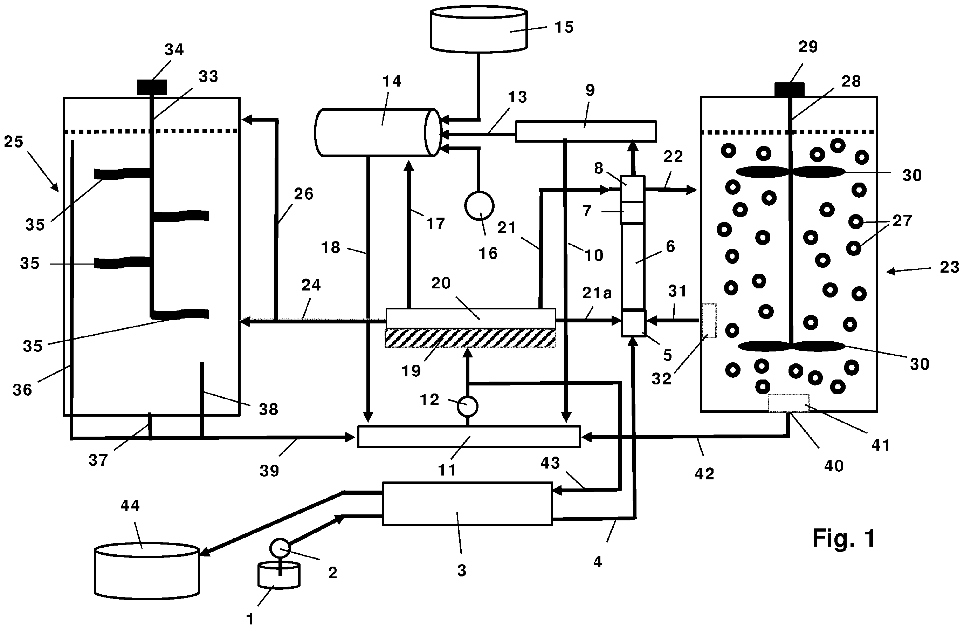

Zunächst soll nachfolgend eine Ausführungsform des erfindungsgemäßen Verfahrens anhand des in Fig. 1 schematisch dargestellten Verfahrensfließbildes, das spezifisch und beispielhaft auf die Verarbeitung von Gülle als Ausgangsmaterial Bezug nimmt, erläutert werden:

Aus einem Güllevorlagebehälter 1, der nicht als Bestandteil einer erfindungsgemäßen Vorrichtung zur Durchführung des Verfahrens angesehen werden muss, pumpt eine Güllezufuhrpumpe 2 als Ausgangsmaterial für die Gewinnung von Biogas verwendete Gülle der Reihe nach durch einen Gegenstromwärmetauscher 3, eine Gülleleitung 4, ein Mehrwegeventil 5, eine Behandlungseinrichtung zum Zerkleinern, Aufbrechen und/oder Homogenisieren eines Feststoffanteils, hier in Form einer elektrokinetischen Desintegrationseinrichtung 6, einen Wärmetauscher 7 und ein Mehrwegeventil 8 bis zu einer Separationseinrichtung 9. Diese separiert den Zustrom an Gülle in einen Dünnstoffanteil, der durch die Effluent-Dünnstoffabflussleitung 10 zu einem saugseitigen Verteilerbalken 11 vor einer Pumpe 12 abfließt, und einen Dickstoffanteil, der über einen Retentat-Dickstoffaustrag 13 in eine Kugelmühle 14 eingebracht wird. In die Kugelmühle 14 kann ggf. zusätzlich aus einem Feststoffvorlagebehälter 15, der eine integrierte Feststoffzerkleinerung enthalten kann, weiteres organisches - ggf. vorzerkleinertes - Ausgangsmaterial, z.B. Mist, Hühnertrockenkot, Stroh, Gras, sonstige pflanzliche Biomasse oder anderes, und/oder aus einer Dosiereinheit für Prozesshilfsmittel 16 z.B. hydrolysefördernde Enzyme, Spurenelemente oder anderes, und/oder über eine Verteilleitung 17 Rezirkulat aus dem Prozess zugegeben werden. Aus der Kugelmühle 14 fließt das Dickstoffmaterial inklusive seiner eventuellen Beimengungen über eine Abflussleitung 18 ebenfalls zum saugseitigen Verteilerbalken 11 vor der Pumpe 12. An die Pumpe 12 schließen sich ein Wärmetauscher 19 sowie ein druckseitiger Verteilerbalken 20 hinter der Pumpe 12 an. Die Pumpe 12 pumpt den Dünnstoffanteil der Reihe nach durch eine Verteilleitung 21, das Mehrwegeventil 8 und eine Verteil- und Umwälzruckflussleitung 22 in einen Dünnstofffermenter 23, der z.B. als Dünnstofffermenterkammer in einem Mehrkammersystem ausgebildet sein kann. Den Dickstoffanteil mit den eventuellen Beimengungen pumpt die Pumpe 12 über eine Verteilleitung 24 in einen Dickstofffermenter 25, der z.B. als Dickstofffermenterkammer in dem Mehrkammersystem ausgebildet sein kann. Über eine weitere Verteilleitung 26 kann der Dickstofffermenter 25 ggf. an alternativer Stelle, u.a. auch mit Rezirkulat aus dem Prozess, gespeist werden. Wenn der Dünnstofffermenter 23 und der Dickstofffermenter 25 auf unterschiedlichen Temperaturen betrieben werden sollen, so können anstelle eines einzigen Wärmetauschers 19 für die Erwärmung der unterschiedlichen Fraktionen (Dickstoffanteil und Dünnstoffanteil) jeweils eigene entsprechend für die Temperierung auf die gewünschten Prozesstemperaturen ausgelegte Wärmetauscher vorgesehen sein. Alternativ können auch in den in den weitere Leitungen (z.B. in den Verteilerleitungen 21 und/oder 21a und oder 24) weitere Heizmittel angeordnet sein.

In dem Dünnstofffermenter 23 sind Aufwuchskörper 27 enthalten, auf denen Mikroorganismen angesiedelt sind, die die organische Fracht der durch den Dünnstoffanteil gebildeten Gärflüssigkeit in dem Dünnstofffermenter 23 zu Biogas verstoffwechseln. Ein Rührwerk 28, das durch einen Rührwerksmotor 29 in Rotation versetzt wird, homogenisiert mit seinen Rührwerksflügeln 30 den Inhalt des Dünnstofffermenters 23 und hält dabei die Aufwuchskörper 27 frei in der Schwebe und über die gesamte Höhe des Dünnstofffermenters 23 gleichmäßig verteilt.

Eine Umwälzabflussleitung 31 aus dem Dünnstofffermenter 23, die mit Hilfe eines Rückhaltesiebes 32 vor dem Eindringen von Aufwuchskörpern 27 geschützt ist, führt über das Mehrwegeventil 5 hin zu der elektrokinetischen Desintegrationseinrichtung 6. Bei der (externen) Umwälzung strömt Gärflüssigkeit im Anschluss über den Wärmetauscher 7, das Mehrwegeventil 8 und die Verteil- und Umwälzrückflussleitung 22 wieder in den Dünnstofffermenter 23 zurück.

Der Dickstofffermenter 25 enthält ein Mischwerk 33, das von einem Mischwerksmotor 34 in Rotation versetzt wird und mit Mischwerksflügeln 35 den Inhalt des Dickstofffermenters 25 mischt. Aus dem Dickstofffermenter 25 kann Kammerinhalt über drei verschiedene, vor allem in unterschiedlicher Höhe positionierte Auslässe abgezogen werden, nämlich über einen Schwimmschichtabzug 36, einen Sinkschichtabzug 37 und einen Substratflüssigkeitsabzug 38. Alle drei Abzüge münden in eine Verteilleitung 39, die zum saugseitigen Verteilerbalken 11 vor der Pumpe 12 führt. Durch die unterschiedlichen Abzüge des Dickstofffermenters 25 und die spezifische Anlage der Verteilleitungen können verschiedene Operationen ausgeführt werden, nämlich insbesondere, sich verflüssigt habender (hydrolysierter) Inhalt des Dickstofffermenters 25 über die elektrokinetische Desintegrationseinrichtung 6 in den Dünnstofffermenter 23 übergeleitet werden, aber auch Schwimmschichten aus dem Dickstofffermenter 25 abgezogen und ggf. erneut in die Kugelmühle 14 eingetragen werden oder Sinkschichten aus dem Dickstofffermenter 25 abgezogen und entweder über die elektrokinetische Desintegrationseinrichtung 6 in den Dünnstofffermenter 23 übergeleitet werden oder bei Bedarf als Gärrest gänzlich ausgetragen werden.

Der Gärrest des Prozesses im Dünnstofffermenter 23 wird regelmäßig über einen Gärrestabzug 40 aus dem Dünnstofffermenter 23 abgezogen. Die Aufwuchskörper 27 werden dabei durch ein Rückhaltesieb 41 zurückgehalten und nicht mit ausgetragen. Über eine Gärrestleitung 42, den saugseitigen Verteilerbalken 11 und die Pumpe 12 verlässt der Gärrest den Prozess über eine Gärresteleitung 43 in ein Gärrestlager 44, welches nicht zwingend als Bestandteil einer das erfindungsgemäße Verfahren durchführenden Anlage bzw. Vorrichtung anzusehen ist, sondern auch extern gestellt werden kann. Dabei passiert der Gärrest den Gegenstromwärmetauscher 3, um so seine Restwärme für die Vorerwärmung der über die Gülleleitung 4 neu zugeführten Gülle zu nutzen.

Das in Fig. 1 dargestellte Verfahren für die Gewinnung von Biogas arbeitet dabei folgendermaßen:
Die zuströmende Gülle wird in dem Gegenstromwärmetauscher 3 durch Aufnahme von Restwärme des durch die Gärresteleitung 43 abfließenden Gärrestes vorerwärmt und sodann in der elektrokinetischen Desintegrationseinrichtung 6 elektrokinetisch desintegriert. Dabei werden durch die elektrische Hochspannung Partikelagglomerationen aufgelöst und zerkleinert, Zellwände zertrümmert und Zellinhaltsstoffe in Lösung gebracht und für die Vergärung besser verfügbar gemacht. Die Viskosität der Flüssigkeit wird zudem spürbar reduziert. Nach einer weiteren Erwärmung im Wärmetauscher 7 gelangt das Material in die Separationseinrichtung 9. Diese separiert den dünnflüssigen und relativ wenig viskosen Dünnstoffanteil, der einen gegenüber der eingangs eingespeisten Gülle einen reduzierten Feststoffanteil mit überwiegend feineren Feststoffpartikeln und vergleichsweise relativ wenig Trockensubstanz enthält, vom eher breiigen bis stichfesten und relativ viskoseren Dickstoffanteil, der einen gegenüber der eingangs eingespeisten Gülle einen erhöhten Feststoffanteil und überwiegend die groben Feststoffpartikel enthält und dabei vergleichsweise viel Trockensubstanz aufweist. Diese Separation erfolgt durch einen Siebeinsatz in der Separationseinrichtung 9 oder ein ähnliches Separationsmittel mit Durchlassöffnungen von vorgegebener Öffnungsweite. Diese Öffnungsweite kann von 20 *µ*m bis 2 mm betragen, wird in der Regel von 50 *µ*m bis 1 mm, insbesondere von 100 *µ*m bis 500 *µ*m betragen, und besonders bevorzugt von 200 *µ*m bis 300 *µ*m, insbesondere von 200 *µ*m bis 250 *µ*m liegen.

Der Dünnstoffanteil fließt, nachdem er den Siebeinsatz oder das vergleichbare Separationsmittel der Separationseinrichtung 9 passiert hat, über die Effluent-Dünnstoffabflussleitung 10 zum saugseitigen Verteilerbalken 11 vor der Pumpe 12 ab. Über den weiteren Wärmetauscher 19, der das Gärsubstrat auf die angestrebte, vorzugsweise thermophile, Prozesstemperatur bringt, den druckseitigen Verteilerbalken 20 und die Verteilleitungen 21, 22 pumpt die Pumpe 12 den Dünnstoffanteil in den Dünnstofffermenter 23. Dort wird der Zufluss an organischer Nährstofffracht von Mikroorganismen verstoffwechselt und zu Biogas abgebaut. Die Mikroorganismen sind hauptsächlich auf den in der Gärflüssigkeit des Dünnstofffermenters 23 frei schwebenden Aufwuchskörpern 27 lokalisiert. Die Aufwuchskörper 27 werden vom Rührwerk 28 in dem Dünnstofffermenter 23 kontinuierlich frei in Schwebe und weitestgehend gleichmäßig verteilt gehalten. Die Aufwuchskörper 27 ermöglichen den Mikroorganismen ein Vielfaches an Populationsdichte sowie ständige bestmögliche räumliche Nähe zur Nahrung und damit letztlich eine drastisch gesteigerte Intensität des Abbaus der organischen Fracht zu Biogas. Um zu Beginn der Inbetriebnahme des Prozesses die Hochfahrzeit des Dünnstofffermenters 23 sehr erheblich zu reduzieren, wird dieser zu Prozessbeginn mit bereits mit Bakterien angeimpften Aufwuchskörpern 27 befüllt. Sofern die elektrokinetische Desintegrationseinrichtung 6 nicht durch Zuführung neuer Gülle (oder anderen neuen Ausgangsmaterials) oder durch Überleitung von Substrat aus dem Dickstofffermenter 25 in den Dünnstofffermenter 23 belegt ist, zirkuliert ein kontinuierlicher (externer) Umwälzstrom des in dem Dünnstofffermenter 23 enthaltenen Gärsubstrats über das Rückhaltesieb 32 und die Umwälzabflussleitung 31 durch die elektrokinetische Desintegrationseinrichtung 6 und dann über die Verteil- und Umwälzrückflussleitung 22 in den Dünnstofffermenter 23 zurück. Der Fluss des (externen) Umwälzstroms wird durch die vom Rührwerk 28 erzeugte vertikale Auftriebswirkung in dem Dünnstofffermenter 23 unterhalten und wird ggf. durch eine in Fig. 1 nicht dargestellte Umwälzpumpe unterstützt. Die, von Unterbrechungen abgesehen, kontinuierliche Zirkulation ermöglicht, dass innerhalb kurzer Zeit ein großer Volumenstrom (ggf. auch mehrfach) einer elektrokinetischen Desintegration unterzogen und damit ein maximaler Aufschluss des Substrats für einen möglichst vollständigen und möglichst schnellen Abbau der organischen Fracht in dem Dünnstofffermenter 23 erreicht werden kann. Das Rückhaltesieb 32 gewährleistet dabei, dass die Aufwuchskörper 27 nicht mit der Gärflüssigkeit mitzirkulieren. Andernfalls würden die auf den Aufwuchskörpern 27 aufsitzenden Mikroorganismen mit desintegriert und somit durch die elektrische Hochspannung in kontraproduktiver Weise abgetötet.

Der in der Separationseinrichtung von dem Siebeinsatz oder dem vergleichbaren Separationsmittel zurückgehaltene Dickstoffanteil wird über den Retentat-Dickstoffaustrag 13 aus der Separationseinrichtung 9 in die Kugelmühle 14 ausgetragen. Der Kugelmühle 14 können zusätzlich aus dem Feststoffvorlagebehälter 15 weitere - ggf. vorzerkleinerte - Feststoffe, wie z.B. Mist, Hühnertrockenkot, Stroh, Gras, sonstige pflanzliche Biomasse oder anderes, und/oder aus der Dosiereinheit für Prozesshilfsmittel 16 z.B. hydrolysefördernde Enzyme, Spurenelemente oder anderes und/oder über die Verteilleitung 17 Rezirkulat aus dem Prozess zugegeben werden, die dann in den Gärprozess mit einfließen.

In der - vorzugsweise beheizten - Kugelmühle 14 wird der Dickstoffanteil inklusive seiner eventuellen Beimengungen feinzerkleinert, homogenisiert und - wenn die Kugelmühle 14 beheizt ist - weiter erwärmt. Über die Abflussleitung 18 aus der Kugelmühle 14, den saugseitigen Verteilerbalken 11, die Pumpe 12, den Wärmetauscher 19, der das Gärsubstrat auf die vorzugsweise thermophile Prozesstemperatur bringt, den druckseitigen Verteilerbalken 20 und die Verteilleitung 24 gelangt der Dickstoffanteil in den Dickstofffermenter 25. Dieser enthält das Mischwerk 33, das mit seinen Mischwerksflügeln 35 den Inhalt des Dickstofffermenters 25 in festgelegten zeitlichen Abständen durchmischt. In dem Dickstofffermenter 25 wird der Dickstoffanteil von entsprechenden Mikroorganismen hydrolysiert, versäuert und teilweise (vor-)vergoren. Auch hier entsteht bereits Biogas. Nach und nach lösen sich die Feststoffe in dem Dickstofffermenter 25 auf, der breiige Inhalt verflüssigt sich zunehmend. Der verflüssigte Teil setzt sich in dem Dickstofffermenter 25 überwiegend in dem unteren Teil ab. Er führt noch immer einen nennenswerten Anteil, allerdings überwiegend kleinerer, partikulärer Stoffe mit sich und wird über den Substratflüssigkeitsabzug 38 aus dem Dickstofffermenter 25 abgezogen und sodann über die Verteilleitung 39, den saugseitigen Verteilerbalken 11, die Pumpe 12, den Wärmetauscher 19, den druckseitigen Verteilerbalken 20, eine Verteilleitung 21a, die elektrokinetische Desintegrationseinrichtung 6 und die Verteil- und Umwälzrückführleitung 22 in den Dünnstofffermenter 23 übergeleitet, damit die Inhaltsstoffe dort endgültig und weitestgehend vollständig vergären. Die Durchleitung durch die elektrokinetische Desintegrationseinrichtung 6 erfolgt, um die verbleibenden Partikel elektrokinetisch zu desintegrieren und damit für einen möglichst vollständigen und möglichst schnellen Abbau in dem Dünnstofffermenter 23 bestmöglich aufzuschließen. Gärreste werden regelmäßig aus dem Dünnstofffermenter 23 entnommen.

Das in dem Gärresteabzug 40 angeordnete Rückhaltesieb 41 verhindert ein Ausschwemmen von Aufwuchskörpern 27 und damit Mikroorganismen. Über die Gärresteleitung 42, den saugseitigen Verteilerbalken 11und die Pumpe 12 verlässt der Gärrest den Prozess über die Gärresteleitung 43 in das Gärrestelager 44. Dabei passiert der Gärrest den Gegenstromwärmetauscher 3, um so Restwärme der Gärreste für die Vorerwärmung der über die Gülleleitung 4 neu zugeführten Gülle zu nutzen.

Das Besondere in dem in Fig. 1 in einem Ausführungsbeispiel dargestellten Verfahren besteht nun darin, dass über die Separationseinrichtung 9 das zulaufende, pumpfähige organische Material zunächst in einen Dünnstoffanteil und einen Dickstoffanteil aufgeteilt wird. So kann der Dickstoffanteil in dem Dickstofffermenter 25 verflüssigt, versäuert und (vor-)vergoren werden. Der Dünnstoffanteil wird dem Dünnstofffermenter 23 zugetragen, wo er seine besondere Behandlung erfährt. Dabei ist weiterhin der Umstand besonders, dass in dem Dünnstofffermenter 23 Aufwuchskörper 27 Anwendung finden, so dass - insbesondere in Kombination mit der mit großem Volumenstrom kontinuierlich durchgeführten elektrokinetischen Desintegration - etwaige Agglomerationen in dem Dünnstofffermenter aufgelöst werden können, zudem durch die Desintegration die für die Umsetzung durch die Mikroorganismen vorhandene Biomasse noch besser aufgeschlossen und entsprechend effizienter durch die Mikroorganismen verstoffwechselt werden kann.

Dabei ist noch einmal zu betonen, dass das erfindungsgemäße Verfahren dann, wenn das Ausgangsmaterial bereits die an den Dünnstoffanteil zu stellenden Bedingungen, insbesondere im Hinblick auf die Partikelgröße, erfüllt (wie z.B. im Falle von Zuchtsauengülle, Fisch- oder Krustentiergülle aus Aquakulturen oder ggfs. auch Molke) eine Separation in Dünnstoff- und Dickstoffanteil entfallen und allein eine Behandlung des Dünnstoffanteils durchgeführt werden kann. Auch die Vorrichtung wird dann durch Wegfall der entsprechenden für die Separation und die Behandlung des Dickstoffanteils vorgesehenen Elemente und Einrichtungen vereinfacht werden können.

Für eine weitere Betrachtung der Wirksamkeit und Effizienz des erfindungsgemäßen Verfahrens wurden Vergleichsbetrachtungen für einen landwirtschaftlichen Betrieb angestellt, bei dem täglich z.B. ca. 32 m³ Rindergülle anfallen, die etwa 7,5 Gew.-% Trockensubstanz enthält. Wird diese Menge an Rindergülle in eine herkömmliche Biogasanlage mit en bloc Fermentierung der Rindergülle eingespeist, so verweilt sie dort etwa 30 Tage. Damit ist ein Fermentervolumen von 30 x 32 m³ vorzusehen, mithin 960 m³. Wird diese Menge an Rindergülle (32 m³) nach der erfindungsgemäßen Vorgehensweise zunächst in einen Dünnstoff- und einen Dickstoffanteil separiert, so werden ca. 27 m³ Dünnstoffanteil mit einem Trockensubstanzgehalt von ca. 5 Gew.-% erhalten und entsprechend ca. 5 m³ Dickstoffanteil, der dann einen Trockensubstanzgehalt von ca. 21 Gew.-% aufweist. In den mit Vorteil vorgesehenen Dickstofffermenter überführt, verweilt der Dickstoffanteil dort etwa 20 Tage, während der Dünnstoffanteil in dem Dünnstofffermenter bereits innerhalb eines Tages vergoren werden kann. Berücksichtigt man dabei auch den zusätzlich in dem Dünnstofffermenter zu fermentierenden Austrag aus dem Dickstofffermenter, so ergibt sich dennoch nur ein benötigtes Faulraumvolumen insgesamt (also für Dickstofffermenter und Dünnstofffermenter zusammengenommen) von 132 m³, und somit eine ganz erhebliche Verringerung. Die Faulraumproduktivität (m³ Biogas pro m³ zur Verfügung stehendem Faulraumvolumen) des erfindungsgemäßen Verfahrens liegt mithin um ein Mehrfaches höher als in herkömmlichen Biogasanlagen.

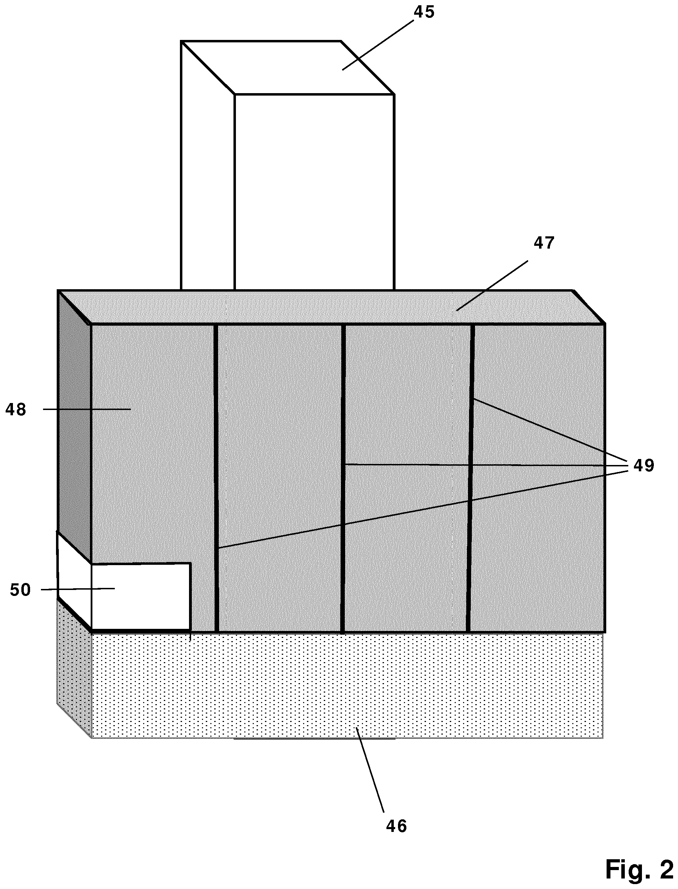

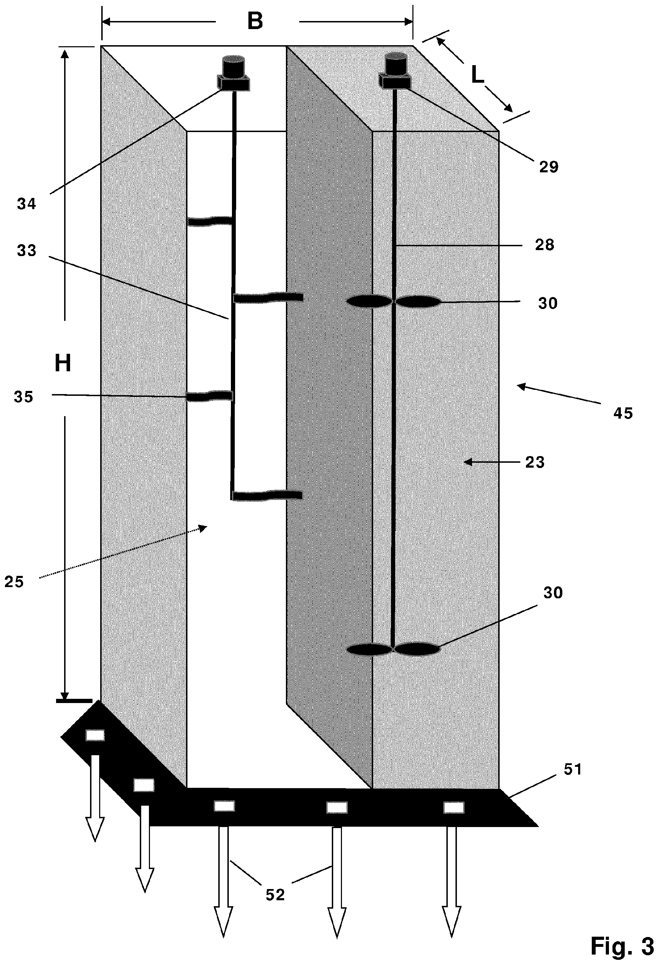

In Fig. 2 ist schematisch der grundsätzliche Aufbau einer erfindungsgemäßen Vorrichtung für die Erzeugung von Biogas in einem Ausführungsbeispiel gezeigt. Dieser Aufbau ist modulartig mit einem im Betrieb vertikal aufgestellten Fermentermodul 45 sowie einem die Abmessungen eines herkömmlichen 40-Fuß-Containers aufweisenden Technikmodul 46. In dem Fermentermodul 45 sind, wie nachfolgend anhand der Fig. 3 noch näher beschrieben, die beiden hier vorgesehenen Fermenter, Dickstofffermenter und Dünnstofffermenter, ausgebildet als Fermenterkammern, untergebracht. Das Technikmodul 46 umfasst den größten Teil der weiteren technischen Einrichtungen und Rohrleitungen, insbesondere die in Fig. 1 mit den Bezugszeichen 3 bis 22, 24, 26, 31, 39, 42 und 43 versehenen Elemente. Die Rohrleitungen des Technikmoduls 46, die in das Modul hineinkommen oder es verlassen, enden an Flanschen, die jeweils an einen Gegenflansch des Fermentermoduls 45 oder an Gegenflansche von bauseitigen Vorrichtungen und/oder bauseitigen Rohrleitungen angekoppelt werden. Das Technikmodul 46 enthält darüber hinaus ein öffen- sowie höhenverfahrbares Dach 47. Dieses öffen- sowie höhenverfahrbare Dach 47 kann mittels einer Seilzugvorrichtung (in Fig. 2 nicht dargestellt) oder hydraulisch oder mit einem anderen Antrieb in der Höhe variabel so ausgefahren werden, wie es dem jeweiligen Füllstand eines ebenfalls in dem Technikmodul 46 integrierten Biogas-Speichersacks 48 entspricht. Höhenverfahrbare Seitenbegrenzungen 49 auf beiden Längsseiten des Technikmoduls 46 begrenzen die Ausdehnung des Biogas-Speichersacks 48. Der Biogas-Speichersack 48 weist eine Aussparung 50 auf, die dazu dient, den im Technikmodul 46 befindlichen Feststoffvorlagebehälter 15, z.B. per Radlader, von oben an dem Biogasspeichersack 48 vorbei befüllen zu können. Alternativ kann der Biogas-Speichersack 48 in einem weiteren eigenen, hier nicht dargestellten Gasspeichermodul, das ebenfalls die Abmessungen eines herkömmlichen 40-Fuß-Containers aufweisen und ggf. auf das Technikmodul 46 aufgestapelt werden kann, untergebracht werden.

In diesem Zusammenhang ist darauf zu verweisen, dass die oben beschriebene Konstruktion eines Aufnahmemoduls für einen Biogas-Speichersack, welches ein öffen- und höhenverfahrbares Dach aufweist, um der Ausdehnung des Biogas-Speichersacks bei zunehmenden Füllstand (Füllvolumen) Rechnung zu tragen, als eigenständige Problemlösung und mithin separate Erfindung gesehen werden kann. Dies gilt insbesondere auch für die Kombination dieser Merkmale mit einem Technikmodul oder einem anderen Modul, das die Abmessungen eines herkömmlichen 40-Fuß-Containers aufweist und z.B. eine äußere Hülle aus Stahl aufweisen kann.

In Fig. 3 ist noch einmal das Fermentermodul 45 dargestellt und das darin befindliche Innenleben skizziert. Hier ist zu erkennen, dass darin der Dünnstofffermenter 23 und der Dickstofffermenter 25 als nebeneinander angeordnete Fermenterkammern mit den jeweiligen Rühr- 28 bzw. Mischwerken 33 und deren Antriebsmotoren 29 und 34 aufgenommen sind. Die jeweilige Breite und damit das jeweilige Fassungsvermögen der beiden Kammern können dabei durchaus unterschiedlich sein. Das Fermentermodul 45 beinhaltet ein Fertigfundament 51, das mit speziellen Fundamentschrauben 52 (z.B. solchen, wie sie von der Firma Krinner Schraubfundamente GmbH aus Straßkirchen, Deutschland, angeboten werden) rasch und effizient einfach an den Untergrund angeschraubt werden kann. Dies ermöglicht nicht nur die Aufstellung des Fermentermoduls 45 ohne aufwendige Vorbereitungen innerhalb kürzester Zeit, sondern auch den einfachen und schnellen Rückbau. Das Fermentermodul 45 ist darüberhinaus mit einer im Fundamentbereich integrierten Leckageerkennung (in Fig. 3 nicht dargestellt) ausgestattet. Das hier skizzierte Fermentermodul 45 hat in der Realität eine Höhe H von ca. 15,00 m, eine Breite B von ca. 3,65 m und eine der Breite identisch entsprechende Länge L von ebenfalls ca. 3,65 m (jeweils ohne eine in der Regel noch aufgebrachte Außendämmung und ohne eine ebenfalls noch aufgebrachte Wetterschutzhaut sowie ohne Fertigfundament).

Eine Besonderheit der in Figuren 2 und 3 skizzierten erfindungsgemäßen Vorrichtung besteht insbesondere darin, dass diese mobil transportabel und an einem Aufstellungsort aufbaubar, ebenso gut aber auch wieder abbaubar und damit umzugsfähig ist. Dies ermöglicht den jeweiligen Aufstellern und Nutzern eine andere Form der Verwendung, z.B. die Möglichkeit, entsprechende Anlagen für bestimmte Zeiten zu mieten, zu leasen oder aber auch käuflich anzuschaffen und bei nicht mehr fortgeführter Nutzung wieder zu veräußern. Der Transport zu dem Aufstellungsgelände kann dabei mit üblichem Schwerlast-Lastkraftwagen erfolgen, wobei die Module Technikmodul 46 und optional Gasspeichermodul (nicht gezeigt) mit normalen Containertransport-Lastkraftwagen verfrachtet werden können, lediglich das Fermentermodul 45 mit Überbreite von einem Spezialfahrzeug, z.B. einem speziell ausgestatteten Tieflader, zu transportieren ist.

Aus der voranstehenden Beschreibung der Ausführungsbeispiele ergibt sich noch einmal deutlich die Besonderheit des erfindungsgemäßen Verfahrens und der erfindungsgemäßen Vorrichtung gegenüber dem Stand der Technik. Die damit verbundenen Vorteile lassen sich daraus klar ablesen.

### Bezugszeichenliste

- 1: Güllevorlagebehälter
- 2: Güllezuführpumpe
- 3: Gegenstromwärmetauscher
- 4: Gülleleitung
- 5: Mehrwegeventil
- 6: elektrokinetische Desintegrationseinrichtung
- 7: Wärmetauscher
- 8: Mehrwegeventil
- 9: Separationseinrichtung
- 10: Effluent-Dünnstoffabflussleitung
- 11: saugseitiger Verteilerbalken
- 12: Pumpe
- 13: Retentat-Dickstoffaustrag
- 14: Kugelmühle
- 15: Feststoffvorlagebehälter
- 16: Dosiereinheit für Prozesshilfsmittel
- 17: Verteilleitung
- 18: Abflussleitung
- 19: Wärmetauscher
- 20: druckseitiger Verteilerbalken
- 21: Verteilleitung
- 21a: Verteilleitung
- 22: Verteil- und Umwälzrückflussleitung
- 23: Dünnstofffermenter
- 24: Verteilleitung
- 25: Dickstofffermenter
- 26: Verteilleitung
- 27: Aufwuchskörper
- 28: Rührwerk
- 29: Rührwerksmotor
- 30: Rührwerksflügel
- 31: Umwälzabflussleitung
- 32: Rückhaltesieb
- 33: Mischwerk
- 34: Mischwerksmotor
- 35: Mischwerksflügel
- 36: Schwimmschichtabzug
- 37: Sinkschichtabzug
- 38: Substratflüssigkeitsabzug
- 39: Verteilleitung
- 40: Gärrestabzug
- 41: Rückhaltesieb
- 42: Gärrestleitung
- 43: Gärrestleitung
- 44: Gärrestlager
- 45: Fermentermodul
- 46: Technikmodul
- 47: Dach
- 48: Biogas-Speichersack
- 49: Seitenbegrenzungen
- 50: Aussparung
- 51: Fertigfundament
- 52: Fundamentschraube

- B: Breite
- H: Höhe
- L: Länge

## Patentansprüche

1. Verfahren zum Erzeugen von Biogas durch Vergärung von pumpfähigen organischen Ausgangsmaterialien, insbesondere von Güllen und Jauchen aus der Tierhaltung, wobei
- ein Zustrom des organischen Ausgangsmaterials aufgeteilt wird in einen Dickstoffanteil mit gegenüber dem Ausgangsmaterial höherem Feststoffanteil und einen flüssigkeitsdominierten Dünnstoffanteil mit gegenüber dem Ausgangsmaterial geringerem Feststoffanteil,
- der Dünnstoffanteil einem Dünnstofffermenter (23), in dem Mikroorganismen auf in dem Füllvolumen des Dünnstofffermenters (23) in der Schwebe gehaltenen Aufwuchskörpern (27) aufgewachsen sind, zugeführt und dort fermentiert wird,
- in dem Dünnstofffermenter (23) entstandenes Biogas aufgefangen und für eine weitere Verwendung abgeführt wird,
- in dem Dünnstofffermenter (23) entstehende Gärreste abgeführt werden
- ein Anteil des Füllvolumens aus dem Dünnstofffermenter (23) zum Zerkleinern und/oder Aufbrechen und/oder Homogenisieren eines Feststoffanteils durch elektrokinetische Desintegration behandelt wird und nach dieser Behandlung wieder dem restlichen Füllvolumen des Dünnstofffermenters (23) zugegeben wird, wobei die Aufwuchskörper (27) mittels einer Rückhaltevorrichtung in dem Dünnstofffermenter (23) derart zurückgehalten werden, dass sie von der Behandlung ferngehalten werden.

2. Verfahren nach Anspruch 1, **dadurch gekennzeichnet, dass** der Dickstoffanteil einem Dickstofffermenter (25) zugeführt und dort hydrolysiert und ggf. zum Teil bereits fermentiert wird, und dass ein durch Hydrolysierung in dem Dickstofffermenter (25) verflüssigter Anteil aus dem Dickstofffermenter (25) abgezogen und in den Dünnstofffermenter (23) überführt und/oder dem Dünnstoffanteil beigemengt und/oder dem Zustrom organischen Ausgangsmaterials zugeleitet wird, wobei vorzugsweise in dem Dickstofffermenter (25) entstehendes Biogas aufgefangen und für eine weitere Verwendung abgeführt wird.

3. Verfahren nach einem der vorhergehenden Ansprüche, **dadurch gekennzeichnet, dass** der Zustrom organischen Ausgangsmaterials vor der Aufteilung in den Dickstoffanteil und den Dünnstoffanteil zum Zerkleinern und/oder Aufbrechen und/oder Homogenisieren der Feststoffe behandelt wird.

4. Verfahren nach Anspruch 3, **dadurch gekennzeichnet, dass** die Behandlung des Zustroms organischen Ausgangsmaterials vor der Aufteilung in den Dickstoffanteil und den Dünnstoffanteil zum Zerkleinern und/oder Aufbrechen und/oder Homogenisieren der Feststoffe durch elektrokinetische Desintegration erfolgt.

5. Verfahren nach einem der vorhergehenden Ansprüche, **dadurch gekennzeichnet, dass** der Zustrom organischen Materials über Wärmetausch mit abströmenden Gärresten vorerwärmt wird.

6. Verfahren nach einem der vorhergehenden Ansprüche, **dadurch gekennzeichnet, dass** der Dünnstoffanteil das Effluent der Separationseinrichtung ist und dass der Dickstoffanteil das Retentat der Separationseinrichtung ist, wobei die Separationseinrichtung einen Siebeinsatz oder ein vergleichbares Separationsmittel mit Durchlassöffnungen mit Öffnungsweiten von 20 *µ*m bis 2 mm aufweist.

7. Verfahren nach einem der vorhergehenden Ansprüche, **dadurch gekennzeichnet, dass** dem Zustrom organischen Ausgangsmaterials gezielt vorzerkleinerte organische Feststoffe zugesetzt werden.

8. Verfahren nach einem der vorhergehenden Ansprüche, **dadurch gekennzeichnet, dass** als organisches Ausgangsmaterial eine Gärsuspension aus einem Fermenter oder einem Nachgärbehälter, auch einem Trockenfermentationsbehälter, einer bestehenden, herkömmlichen Biogasanlage verwendet wird.

9. Vorrichtung zum Erzeugen von Biogas durch Vergärung von pumpfähigen, gegebenenfalls Feststoffe enthaltenden organischen Ausgangsmaterialien, insbesondere von Güllen oder Jauchen aus der Tierhaltung, mit
- einer wenigstens einen Siebeinsatz oder ein vergleichbares Separationsmittel mit Durchlassöffnungen von vorgegebener Öffnungsweite aufweisenden Separationseinrichtung (9) zum Aufteilen von zuströmenden organischen Ausgangsmaterialien in einen als Retentat der Separationseinrichtung gewonnenen Dickstoffanteil und in einen als Effluent der Separationseinrichtung gewonnenen, flüssigkeitsdominerten Dünnstoffanteil,
- einem Dünnstofffermenter (23) zum Fermentieren des Dünnstoffanteils, wobei in dem Dünnstofffermenter (23) Aufwuchskörper (27) für das Aufwachsen von Mikroorganismen angeordnet sind
- einer Behandlungseinrichtung (6) in Form einer elektrokinetischen Desintegration zum Zerkleinern und/oder Aufbrechen und/oder Homogenisieren von in dem Dünnstoffanteil enthaltenen Feststoffen, wobei die Behandlungseinrichtung (6) zum Durchsatz von in dem Dünnstofffermenter (23) enthaltenem Inhalt mit dem Füllvolumen des Dünnstofffermenters (23) in Verbindung steht,
- eine Rückhaltevorrichtung (32) zum Verhindern eines Vordringens der Aufwuchskörper (27) in die Behandlungseinrichtung (6).

10. Vorrichtung nach Anspruch 10, **dadurch gekennzeichnet, dass** der Dünnstofffermenter (23) eine Rühr- und/oder Umwälzeinrichtung (28) zum Umwälzen des Füllvolumens des Dünnstofffermenters (23) und zum in der Schwebe Halten der Aufwuchskörper (27) aufweist

11. Vorrichtung nach einem der Ansprüche 9 oder 10, **gekennzeichnet durch** einen Dickstofffermenter (25) zum Hydrolysieren, Versäuern und Fermentieren des Dickstoffanteils.

12. Vorrichtung nach Anspruch 11, **dadurch gekennzeichnet, dass** der Dickstofffermenter (25) eine Mischeinrichtung (33) aufweist, zum Durchmischen seines Füllvolumens.

13. Vorrichtung nach einem der Ansprüche 9 bis 12, **dadurch gekennzeichnet, dass** die Durchlassöffnungen Öffnungsweiten von 20 *µ*m bis 2 mm, vorzugsweise von 50 *µ*m bis 1 mm, insbesondere von 100 *µ*m bis 300 *µ*m aufweisen.

## Claims

1. A method for producing biogas by fermentation of pumpable organic starting materials, in particular manure and manure from animal husbandry, wherein
- an inflow of the organic starting material is divided into a thick matter fraction with a higher solids content than the starting material and a liquid-dominated thin matter fraction with a lower solids content than the starting material,
the thin material portion is fed to a thin material fermenter (23), in which microorganisms have grown on growth bodies (27) held in suspension in the filling volume of the thin material fermenter (23), and is fermented there,
- biogas produced in the thin material fermenter (23) is collected and discharged for further use,
fermentation residues produced in the thin material fermenter (23) are removed, a portion of the filling volume is treated from the thin material fermenter (23) by electrokinetic disintegration for comminution and/or breaking up and/or homogenisation of a solid fraction and, after this treatment, is added again to the remaining filling volume of the thin material fermenter (23), wherein the growth bodies (27) are retained in the thin material fermenter (23) by means of a retention device such that they are kept away from the treatment.

2. A method according to claim 1, **characterised in that** the thick matter fraction is fed to a thick matter fermenter (25) and is hydrolysed there and optionally partially already fermented, and **in that** a portion liquefied by hydrolysis in the thick matter fermenter (25) is drawn off from the thick matter fermenter (25) and transferred into the thin matter fermenter (23) and/or added to the thin matter portion and/or fed to the influx of organic starting material, wherein biogas generated preferably in the thick matter fermenter (25) is collected and discharged for further use.

3. A method according to one of the preceding claims, **characterised in that** the influx of organic starting material is treated before being divided into the thick matter fraction and the thin matter fraction for comminuting and/or breaking up and/or homogenising the solids.

4. A method according to claim 3, **characterised in that** the treatment of the influx of organic starting material is carried out by electrokinetic disin-tegration before the separation into the thick matter fraction and the thin matter fraction for comminuting and/or breaking up and/or homogenising the solids.

5. A method according to one of the preceding claims, **characterised in that** the influx of organic material is preheated by heat exchange with outflowing fermentation residues.

6. A method according to one of the preceding claims, **characterised in that** the proportion of thin substance is the effluent of the separation device and that the thick matter content is the retentate of the separation device, the separation device having a sieve insert or a comparable separation medium with passage openings with opening widths of 20 µm to 2 mm.

7. A method according to one of the preceding claims, **characterised in that** pre-comminuted organic solids are selectively added to the influx of organic starting material.

8. A method according to one of the preceding claims, **characterised in that** the organic starting material used is a fermentation suspension from a fermenter or a secondary fermentation vessel, also a dry fermentation vessel, of an existing, conventional biogas plant.

9. A device for producing biogas by fermentation of pumpable organic starting materials which may contain solids, in particular slurry or manure from animal husbandry, with a separation device (9) having at least one sieve insert or a comparable separation means with passage openings of predetermined opening width for dividing incoming organic starting materials into a thick matter fraction obtained as retentate of the separation device and into a liquid-dominated thin matter fraction obtained as effluent of the separation device, **characterised in that** the separation device (9) has at least one sieve insert or a comparable separation means with passage openings of prede-termined opening width,
- a thin material fermenter (23) for fermenting the thin material portion, wherein growth bodies (27) for the growth of microorganisms are arranged in the thin material fermenter (23), a treatment device (6) in the form of an electrokinetic disintegration for comminuting and/or breaking up and/or homogenising solids contained in the thin material portion, the treatment device (6) being connected to the filling volume of the thin material fermenter (23) for throughput of content contained in the thin material fermenter (23),
a retention device (32) for preventing the growth bodies (27) from advancing into the treatment device (6).

10. A device according to claim 10, **characterised in that** the thin material fermenter (23) has a stirring and/or circulating device (28) for circulating the filling volume of the thin material fermenter (23) and for holding the growth bodies (27) in suspension.

11. A device according to one of claims 9 or 10, **characterised by** a thick matter fermenter (25) for hydrolysing, acidifying and fermenting the thick matter fraction.

12. A device according to Claim 11, **characterised in that** the thick matter fermenter (25) has a mixing device (33) for mixing through its filling volume.

13. A device according to one of claims 9 to 12, **characterised in that** the passage openings have opening widths of 20 µm to 2 mm, preferably of 50 µm to 1 mm, in particular of 100 µm to 300 µm.

## Revendications

1. Procédé de production de biogaz par fermentation de matières premières organiques pompables, en particulier de lisier et de lisier d'élevage, dans lequel
- un afflux du matériau de départ organique est divisé en une fraction de matière épaisse ayant une teneur en solides supérieure à celle du produit de départ et une fraction de matière fine dominée par un liquide ayant une teneur en solides inférieure au matériau de départ,
la partie de matière fine est introduite dans un fermenteur de matière fine (23) dans lequel des microorganismes se sont développés sur des corps de croissance (27) maintenus en suspension dans le volume de remplissage du fermenteur de matière fine (23) et y est fermentée,
- le biogaz produit dans le fermenteur de matière fine (23) est collecté et évacué pour une utilisation ultérieure,
les résidus de fermentation produits dans le fermenteur de matière fine (23) sont éliminés, une partie du volume de remplissage est traitée à partir du fermenteur de matière fine (23) par désintégration électrocinétique en vue de la pulvérisation et/ou de la fragmentation et/ou de l'homogénéisation d'une fraction solide et, après ce traitement, est ajouté à nouveau au volume de remplissage restant du fermenteur de matière fine (23), dans lequel les corps de croissance (27) sont retenus dans le fermenteur de matière fine (23) au moyen d'un dispositif de rétention de telle sorte qu'ils soient tenus à l'écart du traitement.

2. Procédé selon la revendication 1, **caractérisé en ce que** la fraction de matière épaisse est introduite dans un fermenteur de matière épaisse (25), y est hydrolysée et éventuellement déjà partiellement fermentée et **en ce qu'**une partie liquéfiée par hydrolyse dans le fermenteur de matière épaisse (25) est extraite du fermenteur de matière épaisse (25) et transférée dans le fermenteur de matière fine (23) et/ou ajoutée à la partie de matière fine et/ou alimentée à l'afflux de matière première organique, dans lequel le biogaz généré de préférence dans le fermenteur de matière épaisse (25) est collecté et évacué pour une utilisation ultérieure.

3. Procédé selon l'une des revendications précédentes, **caractérisé en ce que** l'afflux de produit de départ organique est traité avant d'être divisé en fraction de matière épaisse et fraction de matière fine pour pulvériser et/ou fracturer et/ou homogénéiser les solides.

4. Procédé selon la revendication 3, **caractérisé en ce que** le traitement de l'afflux de matière première organique est réalisé par désintégration électrocinétique avant séparation dans la fraction de matière épaisse et la fraction de matière fine pour la pulvérisation et/ou la fracturation et/ou l'homogénéisation de solides.

5. Procédé selon l'une des revendications précédentes, **caractérisé en ce que** l'afflux de matière organique est préchauffé par échange de chaleur avec les résidus de fermentation en sortie.

6. Procédé selon l'une quelconque des revendications précédentes, **caractérisé en ce que** la proportion de substance fine est l'effluent du dispositif de séparation et que le contenu en matière épaisse est le rétentat du dispositif de séparation, le dispositif de séparation ayant un insert de tamis ou un moyen de séparation comparable avec des ouvertures de passage avec des largeurs d'ouverture de 20 µm à 2 mm.

7. Procédé selon l'une des revendications précédentes, **caractérisé en ce que** des solides organiques pré-broyés sont ajoutés sélectivement à l'afflux de matériau de départ organique.

8. Procédé selon l'une des revendications précédentes, **caractérisé en ce que** le matériau de départ organique utilisé est une suspension de fermentation provenant d'un fermenteur ou un récipient de fermentation secondaire, également un récipient de fermentation à sec, d'une installation de production de biogaz conventionnelle existante.

9. Dispositif permettant de produire du biogaz par fermentation de produits de départ organiques pompables pouvant contenir des solides, en particulier du lisier ou du lisier d'élevage, avec un dispositif de séparation (9) comportant au moins un tamis ou un moyen de séparation comparable avec des ouvertures de passage prédéterminées largeur d'ouverture pour diviser des matériaux de départ organiques entrants en une fraction de matière épaisse obtenue comme rétentat du dispositif de séparation et en une fraction de matière fine liquide à prédominance obtenu comme effluent du dispositif de séparation, **caractérisé en ce que** le dispositif de séparation (9) présente au moins un insert de tamis ou un moyen de séparation comparable avec des ouvertures de passage d'une largeur d'ouverture prédéterminée,
- un fermenteur de matière fine (23) pour fermenter la partie de matière fine, dans lequel des corps de croissance (27) destinés à la croissance de microorganismes sont disposés dans le fermenteur de matière fine (23), un dispositif de traitement (6) se présentant sous la forme d'une désintégration électrocinétique pour pulvériser et/ou fracturer et/ou homogénéiser les solides contenus dans la partie de matière fine, le dispositif de traitement (6) étant relié au volume de remplissage du fermenteur de matière fine (23) pour permettre le débit du contenu présent dans le fermenteur de matière fine (23),
un dispositif de retenue (32) destiné à empêcher les corps de croissance (27) de progresser dans le dispositif de traitement (6).

10. Dispositif selon la revendication 10, **caractérisé en ce que** le fermenteur de matière fine (23) présente un dispositif de brassage et/ou de circulation (28) pour faire circuler le volume de remplissage du fermenteur de matière fine (23) et pour maintenir les corps de croissance (27) en suspension.

11. Dispositif selon l'une des revendications 9 ou 10, **caractérisé par** un fermenteur de matière épaisse (25) pour hydrolyser, acidifier et fermenter la fraction de matière épaisse.

12. Dispositif selon la revendication 11, **caractérisé en ce que** le fermenteur de matière épaisse (25) comporte un dispositif de mélange (33) destiné à être mélangé à travers son volume de remplissage.

13. Dispositif selon l'une des revendications 9 à 12, **caractérisé en ce que** les ouvertures de passage ont des largeurs d'ouverture de 20 µm à 2 mm, de préférence de 50 µm à 1 mm, en particulier de 100 µm à 300 µm.
